# EUROPEAN PATENT SPECIFICATION

(11) **EP 3 488 007 B1**
(45) Date of publication and mention of the grant of the patent: **20.12.2023**
(21) Application number: 17831956.2
(22) Date of filing: 21.07.2017
(51) Int. Cl.: C12N 15/861, C12N 7/00, A61K 48/00, C07K 2/00, C12N 15/64

(54) **SCALABLE HIGH RECOVERY METHODS FOR PRODUCING HIGH YIELD RECOMBINANT ADENO-ASSOCIATED VIRAL (RAAV) VECTOR AND RECOMBINANT ADENO-ASSOCIATED VIRAL (RAAV) VECTORS PRODUCED THEREBY**
SKALIERBARES HOCHGRADIGES RÜCKGEWINNUNGSVERFAHREN ZUR HERSTELLUNG VON REKOMBINANTEN ADENO-ASSOZIIERTEN VIRALEN (RAAV) VEKTOREN MIT HOHEM ERTRAG UND DAMIT HERGESTELLTE REKOMBINANTE ADENO-ASSOZIIERTE VIRALE (RAAV) VEKTOREN
PROCÉDÉS ÉVOLUTIFS ET À HAUT RENDEMENT POUR LA PRODUCTION DE GRANDES QUANTITÉS DE VECTEUR VIRAL ADÉNO-ASSOCIÉ RECOMBINÉ (VAAR) ET VECTEURS VIRAUX ADÉNO-ASSOCIÉS RECOMBINÉS (VAAR) AINSI PRODUITS

(30) Priority: 21.07.2016 US 201662365312 P
(43) Date of publication of application: 29.05.2019
(73) Proprietor: Spark Therapeutics, Inc., Philadelphia, Pennsylvania 19104 (US)
(72) Inventor: QU, Guang, Sicklerville New Jersey 08081 (US); WRIGHT, John Fraser, Redwood City California 94061 (US); OH, Younghoon, Bryn Mawr Pennsylvania 19010 (US); WANG, Yuhuan, Mount Laurel New Jersey 08054 (US); ZHANG, Haibo, Rutledge Pennsylvania 19070 (US); DUNCAN, Laura, Paoli, Pennsylvania 19301 (US)
(74) Representative: Vossius & Partner Patentanwälte Rechtsanwälte mbB
(86) International application number: PCT/US2017/043291
(87) International publication number: WO 2018/017956

(56) References cited:
- WO-A1-2005/118792
- WO-A1-2011/094198
- WO-A1-2016/128408
- WO-A2-2006/087643
- US-A1- 2011 165 645
- US-A1- 2013 072 548
- US-A1- 2015 275 186
- US-A1- 2016 024 139
- J. WRIGHT: "Product-Related Impurities in Clinical-Grade Recombinant AAV Vectors: Characterization and Risk Assessment", BIOMEDICINES, vol. 2, no. 1, 3 March 2014 (2014-03-03), pages 80-97, XP055533563, DOI: 10.3390/biomedicines2010080
- WRIGHT J FRASER ET AL: "Recombinant adeno-associated virus: formulation challenges and strategies for a gene therapy vector", CURRENT OPINION IN DRUG DISCOVERY AND DEVELOPMENT, CURRENT DRUGS, LONDON, GB, vol. 6, no. 2, 1 March 2003 (2003-03-01), pages 174-178, XP009092903, ISSN: 1367-6733
- GRZENIA D L ET AL: "Tangential flow filtration for virus purification", JOURNAL OF MEMBRANE SCIENCE, ELSEVIER BV, NL, vol. 321, no. 2, 15 August 2008 (2008-08-15), pages 373-380, XP022820479, ISSN: 0376-7388, DOI: 10.1016/J.MEMSCI.2008.05.020 [retrieved on 2008-05-21]

## Description

### INTRODUCTION

Tangential Flow Filtration (TFF) is a technique used in the biotechnology industry to concentrate the products through ultrafiltration process (UF) and for buffer exchange through diafiltration (DF) to formulate the products. However, when TFF is used to do UF/DF for recombinant adeno-associated viral (rAAV) vectors, significant amounts of rAAV vectors were lost in the process. When rAAV vectors are highly concentrated, even more vectors will be lost. The amount of vector lost in the UF/DF process make it almost impossible to make high concentration rAAV vector preps which is highly desired in development of rAAV related gene therapy products.

The other phenomenon often observed in manufacture of rAAV vectors is that the rAAV vectors tend to get aggregated in the UF/DF process, particularly when the preps of vectors are highly concentrated, such as 1E+13 vg per ml (1×10¹³ vector genomes/ml; vg: vector genome) or higher. The rAAV vector aggregation seems titer dependent, the higher the titer, and the more aggregation of vector. The vector aggregation also results in vector loss during manufacture. In addition, the aggregated vectors are a concern for patient safety when used in clinics.

The problems with rAAV vector aggregation and vector loss in the UF/DF process using TFF techniques described above represent significant process challenges in the manufacture and/or purification of rAAV vectors. The invention herein addresses these problems.

### SUMMARY

Out of many different hypothesis, rAAV vector aggregation and vector loss in the UF/DF process using TFF techniques may be process dependent and/or formulation dependent. Based on such a hypothesis, new formulations were designed and new operation parameters created for the UF/DF process to improve vector recovery and reduce, minimize or prevent rAAV vector from aggregation. In invention formulations and operation parameters for the UF/DF process, rAAV vector recovery improved and rAAV vector aggregation reduced. The data disclosed herein show that the new formulations and operation parameters created for the UF/DF process, alone or in combination, provide significant increases in the overal rAAV vector recovered from the initial rAAV vector preparation. In fact, rAAV vectors can be concentrated to 5E+13 vg/ml (5×10¹³ vector genomes/ml) without significant vector loss, and the vectors are predominantly monomers, indicating reductions in aggregation.

Invention formulations and/or operation parameters disclosed herein will enable concentration of rAAV vectors to a high concentration, for example, up to 5E+13 (5×10¹³) vector genomes per milliliter (Vg/ml) with little if any rAAV aggregates. In addition, invention formulations and/or operation parameters disclosed herein will improve rAAV vector recovery from buffer exchange or concentrate the vectors using tangential flow filtration techniques (TFF). Invention formulations and/or operation parameters disclosed herein can support any gene therapy clinical application using rAAV vectors, such as rAAV mediated gene therapy for lysosomal storage diseases, such as Neuronal Ceroid-Lipofuscinosis 2 (CLN2), rAAV mediated gene therapy for blood clotting deficiencies (Hemophilia A and Hemophilia B) and so on, when high concentration of rAAV preparations are desired. The improved vector recovery through ultrafitration (UF) and diafiltration (DF) process using TFF will also reduce cost of goods of rAAV vector manufacturing, and also provide a more robust and consistent rAAV vectors or clinical applications.

Accordingly, the invention provides various benefits to the rAAV manufacturing process. In one embodiment, rAAV vector recovery is enhanced, and can be enhanced significantly, thereby providing improved yield from start to finish (e.g., 70% or greater r AAV vector yield from start to finish). In another embodiment, rAAV aggregation is reduced, minimized or prevented, particularly when rAAV vector is concentrated to high rAAV vector titers. In a further embodiment, rAAV vector is produced at high concentration (e.g., at least at 1E+12, 5E+12, 1E+13, or 5E+13 vg/ml), suitable for clinical applications where high titer and/or small volume of administration (e.g., injection or infusion) of rAAV vector is desired. In additional embodiments, the invention provides one or more of the following, alone, or in any combination with each other, or in any combination with the other embodiments disclosed herein:
(1) A highly reliable/consistent UF/DF process for manufacturing rAAV vectors, which can reduce manufacturing risks based upon operation variability;
(2) Efficient rAAV vector recovery from the UF/DF process, providing an overall increase in rAAV vector yield (e.g., 70% or greater from start to finish);
(3) Avoid significant rAAV vector loss;
(4) Reduce, minimize or prevent rAAV vector aggregation (e.g., rAAV vector aggregation not detectable by techniques such as size exclusion chromatography and/or dynamic light scattering);
(5) Allowing concentration of rAAV vector to high concentration, for example, 5E+13 vg/ml with reduced, minimized or little or no detectable rAAV vector aggregation (by techniques such as size exclusion chromatography and/or dynamic light scattering); and/or
(6) Providing high titer rAAV vector preparations to enable small volume administration in clinical applications.

### DESCRIPTION OF DRAWINGS

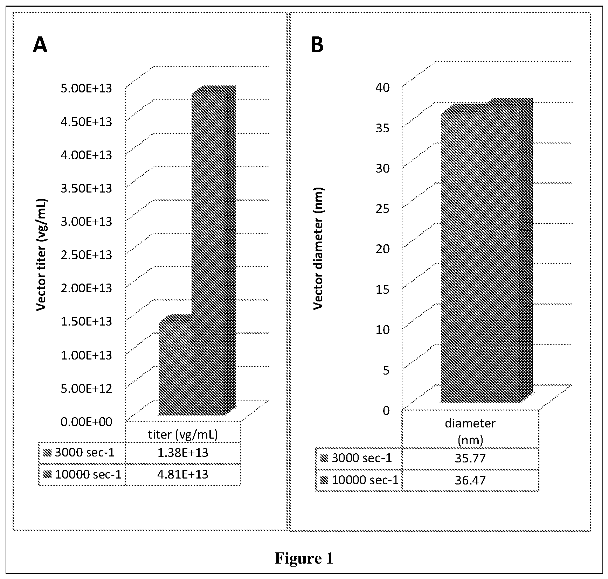

**Figure 1** **shows** how an increase of shear rate during TFF process significantly improved rAAV vector recovery. AAV2-hTPP1v1 vectors (2.5E+14 vg) of post CsCl ultracentrifugation were initially diluted to 50 mL (5.0E+12 vg/mL) using diafiltration buffer. For one cycle of diafiltration, 25 mL diafiltration buffer was added into the 50 mL diluted vectors and then began diafiltration until the volume decreased to 50 mL. The diafiltration step was repeated 24 times. The vectors were ultrafiltrated to 5 mL following the 25 cycles of diafiltration process. The 5 mL vectors were collected for assays. (A) qPCR analysis showed that the vector titer was 4.81E+13 vg/mL when a higher shear rate (∼10,000 sec-1) was employed during TFF process, suggesting more than 90% vector recovery; while 1.38E+13 vg/mL was achieved at a low shear rate (∼3000 sec-1), representing about 30% vector recovery. (B) Dynamic light scattering (DLS) analysis showed that the diameters of vectors from either higher shear rate (∼10,000 sec-1) or low shear rate (∼3000 sec-1), were similar.

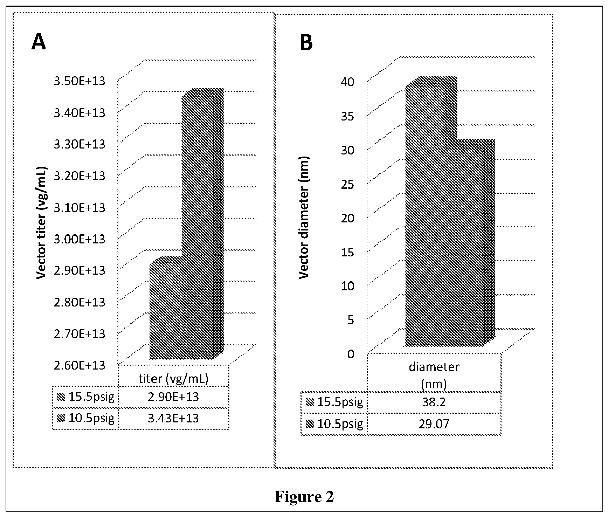

**Figure 2** **shows** how trans-membrane pressure (TMP) affects rAAV recovery from TFF process. AAV2-hTPP1v1 vectors (2.5E+14 vg) of post CsCl ultracentrifugation were initially diluted to 50 mL (5.0E+12 vg/mL) using diafiltration buffer and DF process started, for each diafiltration cycle, 25 mL diafiltration buffer was added into the 50 mL diluted vectors and the volume was reduced to 50 ml. The diafiltration step was performed 25 times to achieve sufficient buffer exchange. The vectors were then ultrafiltrated to 5 mL. Q-PCR analysis (A) showed that the vector titer was 3.43E+13 vg/mL when TMP (∼10.5 psig) was employed and the titer was 2.90E+13 vg/mL in higher TMP (∼15.5 psig). DLS analysis showed that the diameters of vector preps are 29.07 nm and 38,2 nm for the low TMP (∼10 psig) and relative high TMP (∼15.5 psig), respectively. The larger diameter represents more aggregated rAAV vectors in the preparation.

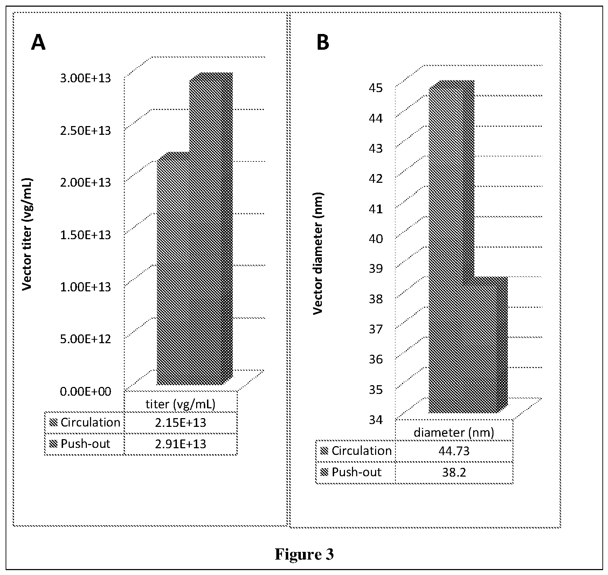

**Figure 3** **shows** data indicating that collection of vectors without circulation post UF/DF process avoids recovery of aggregated rAAV particles in the vector preparation. AAV2-hTPP1v1 vectors (2.5E+14 vg) of post CsCl ultracentrifugation were diluted to 50 mL (5.0E+12 vg/mL) using diafiltration buffer and went through UF/DF process. The vectors were collected either by directly draining out the concentrated vectors (push-out) or by draining out the concentrated vectors after several minutes cyculation (circulation), with lower than UF/DF flow rate. Q-PCR analysis (A) showed that the vector titers were similar with two different vector recovery methods. However, DLS analysis (B) showed that the diameter of vectors was significantly larger in the circulation prep, suggesting more aggregated rAAV vectors.

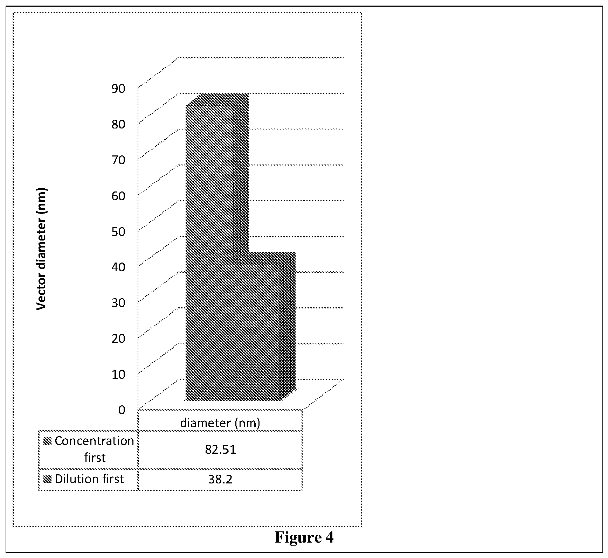

**Figure 4** **shows** data indicating that diluting low titer vector (∼5E+12 vg/mL) substantially reduced the vector aggregation through UF/DF process. AAV2-hTPP1v1 vectors (2.5E+14 vg) of post CsCl ultracentrifugation were either diafiltrated directly or diluted first using diafiltration buffer, then diafiltrated for 25 cycles. Both of the diafiltrated vectors were ultrafiltrated to 5 mL following the diafiltration process. DLS analysis showed that the diameter of vectors (left bar) recovered from dialfiltration process without dilution was significantly larger than that of vectors (right bar) with dilution.

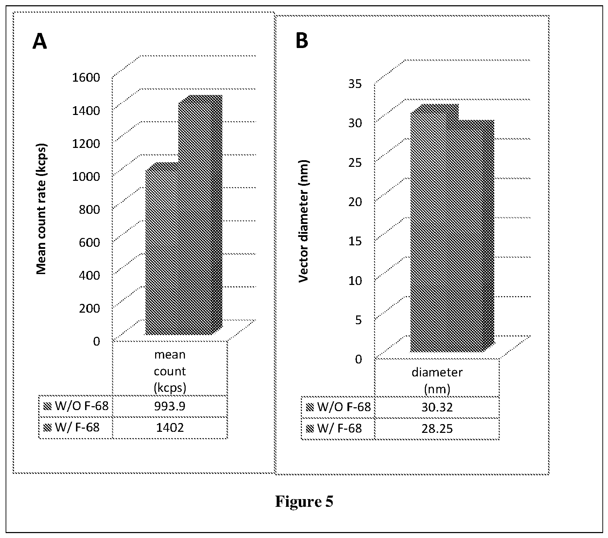

**Figure 5** **shows** data indicating that Pluronic F-68 reduces, minimizes or prevents rAAV vectors forming larger particles (i.e., aggregates). AAV2-hTPP1v1 empty particles of post CsCl ultracentrifugation, as model viral particles, were initially diluted with diafiltration buffer containing pluronic F-68 (named as buffer 1) or no pluronic F-68 (named as buffer 2). The diluted empty particles were then diafiltrated for 25 cycles with either diafiltration buffer 1 or buffer 2 for every cycle. The empty particles were ultrafiltrated to 5 mL following the diafiltration process. The 5 mL empty particles were collected for assays. (A) DLS analysis showed that the mean count (kcps) of empty particles with pluronic F-68 (right bar) was significantly higher than that without pluronic F-68 (left bar), suggesting that better recovery was achieved in the presence of pluronic F-68. (B) The diameter of empty particles without pluronic F-68 (left bar) were larger than that of empty particles with pluronic F-68 (left bar).

### DETAILED DESCRIPTION

Tangential Flow Filtration is widely used technique to do buffer exchange (diafiltration, DF) and to concentrate (ultrafiltration, UF) for bio-products. However, when the TFF technique was used for rAAV vector buffer exchange and final formulation, it was found that a significant amount of vectors were lost in the UF/DF process. High rAAV vector titer/concentration is often needed to treat certain human diseases. However, the current formulation and operation parameters for UF/DF could not produce rAAV vector to the concentration desired, such as vector titer of 1E+13 vg/ml or higher. In addition, rAAV vectors tend to be aggregated when concentrated to the level of 1E+13vg/ml or higher.

In order to overcome these substantial technical limitations to manufacturing high titer rAAV vectors, in this invention a new formulation for the TFF process of rAAV vectors was created. In addition, in this invention new operation parameters for the TFF process were created. When the newly developed formulation was used in the newly created TFF process (with the newly developed parameters) to go through the final UF/DF process to formulate the rAAV vector preps, it was found that the rAAV vector recovery is significantly improved and the rAAV vectors remain as monomers. In particular, with the new TFF formulation in combination with the new TFF operation technique, rAAV vectors were concentrated without substantial rAAV vector aggregation, even when concentrated up to 5E+13 vg/ml. In addition, rAAV vector recovery (overall yield) was significantly improved.

This invention provides a solution to be able to concentrate rAAV vector products to high concentration without detectable rAAV vector aggregation, up to 5E+13 vg/ml without detectable rAAV aggregation. This invention also provides a solution to go through UF/DF process using TFF techniques for rAAV vector without losing significant amount of vector, for example, the rAAV vector recovered from the UF/DF process is in the ranges of 70% to up to 95%, from start to finish. This invention enabled TFF as an efficient technique for rAAV manufacture process, particularly when concentrated rAAV vectors are to be manufactured.

The term "vector" refers to small carrier nucleic acid molecule, a plasmid, virus (e.g., AAV vector), or other vehicle that can be manipulated by insertion or incorporation of a nucleic acid. Such vectors can be used for genetic manipulation (*i.e.,* "cloning vectors"), to introduce/transfer polynucleotides into cells, and to transcribe or translate the inserted polynucleotide in cells. An "expression vector" is a specialized vector that contains a gene or nucleic acid sequence with the necessary regulatory regions needed for expression in a host cell. A vector nucleic acid sequence generally contains at least an origin of replication for propagation in a cell and optionally additional elements, such as a transgene, expression control element *(e.g.,* a promoter, enhancer), intron, ITR(s), polyadenylation signal.

A viral vector is derived from or based upon one or more nucleic acid elements that comprise a viral genome. Particular viral vectors include adeno-associated virus (AAV) vectors.

The term "recombinant," as a modifier of vector, such as recombinant viral, e.g., parvo-virus (e.g., rAAV) vectors, as well as a modifier of sequences such as recombinant polynucleotides and polypeptides, means that the compositions have been manipulated (i.e., engineered) in a fashion that generally does not occur in nature. A particular example of a recombinant vector, such as a rAAV vector would be where a polynucleotide that is not normally present in the wild-type viral (e.g., AAV) genome is inserted within the viral genome. Although the term "recombinant" is not always used herein in reference to vectors, such as AAV vectors, as well as sequences such as polynucleotides, recombinant forms are expressly included in spite of any such omission.

A recombinant viral "vector" or "rAAV vector" is derived from the wild type genome of a virus, such as AAV by using molecular methods to remove the wild type genome from the virus (e.g., AAV), and replacing with a non-native nucleic acid. Typically, for AAV one or both inverted terminal repeat (ITR) sequences of AAV genome are retained in the rAAV vector. A "recombinant" viral vector (e.g., rAAV) is distinguished from a viral *(e.g.,* AAV) genome, since all or a part of the viral genome has been replaced with a non-native sequence with respect to the viral *(e.g.,* AAV) genomic nucleic acid. Incorporation of a non-native sequence therefore defines the viral vector (e.g., AAV) as a "recombinant" vector, which in the case of AAV can be referred to as a "rAAV vector."

A recombinant vector can be packaged- referred to herein as a "particle" for subsequent infection (transduction) of a cell, *ex vivo, in vitro* or *in vivo.* Where a recombinant vector sequence is encapsidated or packaged into an AAV particle, the particle can also be referred to as a "rAAV." Such particles include proteins that encapsidate or package the vector genome. Particular examples include viral envelope proteins, and in the case of AAV, capsid proteins.

A vector "genome" or conveniently abbreviated as "vg" refers to the portion of the recombinant plasmid sequence that is ultimately packaged or encapsidated to form a viral (e.g., rAAV) particle. In cases where recombinant plasmids are used to construct or manufacture recombinant vectors, the vector genome does not include the portion of the "plasmid" that does not correspond to the vector genome sequence of the recombinant plasmid. This non vector genome portion of the recombinant plasmid is referred to as the "plasmid backbone," which is important for cloning and amplification of the plasmid, a process that is needed for propagation and recombinant AAV vector production, but is not itself packaged or encapsidated into virus *(e.g.,* AAV) particles. Thus, a vector "genome" refers to the nucleic acid that is packaged or encapsidated by virus *(e.g.,* AAV).

The terms "polynucleotide" and "nucleic acid" are used interchangeably herein to refer to all forms of nucleic acid, oligonucleotides, including deoxyribonucleic acid (DNA) and ribonucleic acid (RNA). Polynucleotides include genomic DNA, cDNA and antisense DNA, and spliced or unspliced mRNA, rRNA tRNA and inhibitory DNA or RNA (RNAi, *e.g.,* small or short hairpin (sh)RNA, microRNA (miRNA), small or short interfering (si)RNA, trans-splicing RNA, or antisense RNA). Polynucleotides include naturally occurring, synthetic, and intentionally modified or altered polynucleotides (e.g., variant nucleic acid). Polynucleotides can be single, double, or triplex, linear or circular, and can be of any length. In discussing polynucleotides, a sequence or structure of a particular polynucleotide may be described herein according to the convention of providing the sequence in the 5' to 3' direction.

A "transgene" is used herein to conveniently refer to a polynucleotide or a nucleic acid that is intended or has been introduced into a cell or organism. Transgenes include any nucleic acid, such as a gene that encodes a polypeptide or protein.

The "polypeptides," "proteins" and "peptides" encoded by the "nucleic acid" or "polynucleotide" sequences" or "transgenes" include full-length native sequences, as with naturally occurring wild-type proteins, as well as functional subsequences, modified forms or sequence variants so long as the subsequence, modified form or variant retain some degree of functionality of the native full-length protein. Such polypeptides, proteins and peptides encoded by the nucleic acid sequences can be but are not required to be identical to the endogenous protein that is defective, or whose expression is insufficient, or deficient in the treated mammal.

Recombinant AAV vector, include any viral strain or serotype. As a non-limiting example, a rAAV vector can be based upon any AAV genome or AAV capsid, such as AAV-1, -2, -3, -4, -5, -6, -7, -8, -9, -10, -11, -12, -rh74, -rh10 or AAV-2i8, for example. Such vectors can be based on the same strain or serotype (or subgroup or variant), or be different from each other. As a non-limiting example, a recombinant AAV vector based upon one serotype genome can be identical to one or more of the ITRs or capsid proteins that package the vector. In addition, a recombinant AAV vector genome can be based upon an AAV (e.g., AAV2) serotype genome distinct from one or more of the AAV ITRs or capsid proteins that package the vector. For example, the AAV vector genome can be based upon AAV2, whereas at least one of the three capsid proteins could be a AAV1, AAV3, AAV4, AAV5, AAV6, AAV7, AAV8, AAV9, AAV10, AAV11, AAV12, Rh10, Rh74 or AAV-2i8 or variant thereof, for example.

In particular embodiments, rAAV vectors include AAV1, AAV2, AAV3, AAV4, AAV5, AAV6, AAV7, AAV8, AAV9, AAV10, AAV11, AAV12, Rh10, Rh74 and AAV-2i8, as well as variants (e.g., ITR and capsid variants, such as amino acid insertions, additions, substitutions and deletions) thereof, for example, as set forth in WO 2013/158879 (International Application PCT/US2013/037170), WO 2015/013313 (International Application PCT/US2014/047670) and US 2013/0059732 (US Application No. 13/594,773, discloses LK01, LK02, LK03, etc.).

AAV variants include variants and chimeras of AAV1, AAV2, AAV3, AAV4, AAV5, AAV6, AAV7, AAV8, AAV9, AAV10, AAV11, AAV12, Rh10, Rh74 and AAV-2i8 ITRs and capsids. Accordingly, AAV vectors and AAV variants (e.g.,ITR and capsid variants) are included.

In various exemplary embodiments, an AAV vector related to a reference serotype has a polynucleotide (e.g., ITR), or polypeptide (e.g., capsid) that includes or consists of a sequence at least 80% or more *(e.g.,* 85%, 90%, 95%, 96%, 97%, 98%, 99%, 99.1%, 99.2%, 99.3%, 99.4%, 99.5%, etc.) identical to one or more AAV1, AAV2, AAV3, AAV4, AAV5, AAV6, AAV7, AAV8, AAV9, AAV10, AAV11, AAV12, Rh10, Rh74 or AAV-2i8 (e.g., such as an ITR, or a VP1, VP2, and/or VP3 sequences).

As used herein the phrase *"bona fide* AAV vector" or *"bona fide* rAAV vector" refers to AAV vectors comprising a transgene of interest which are capable of infecting target cells. The phrase excludes empty AAV vectors (no transgene is present), and AAV vectors lacking full inserts (e.g., transgene fragments) or those AAV vectors containing host cell nucleic acids.

The term "isolated," when used as a modifier of a composition, means that the compositions are made by the hand of man and/or are separated, completely or at least in part, from their naturally occurring *in vivo* environment. Generally, isolated compositions are substantially free of one or more materials with which they normally associate with in nature, for example, one or more protein, nucleic acid, lipid, carbohydrate, cell membrane. For example, an "isolated nucleic acid" may comprise a DNA or cDNA molecule inserted into a vector, such as a rAAV vector.

Unless otherwise defined, all technical and scientific terms used herein have the same meaning as commonly understood by one of ordinary skill in the art to which this invention belongs.

Various terms relating to the biological molecules of the invention are used hereinabove and also throughout the specification and claims.

As used herein, the singular forms "a", "and," and "the" include plural referents unless the context clearly indicates otherwise. Thus, for example, reference to "a nucleic acid" includes a plurality of such nucleic acids, reference to "a vector" includes a plurality of such vectors, and reference to "a virus" or "particle" includes a plurality of such viruses/particles.

As used herein, all numerical values or numerical ranges include integers within such ranges and fractions of the values or the integers within ranges unless the context clearly indicates otherwise. Thus, to illustrate, reference to 80% or more identity, includes 81%, 82%, 83%, 84%, 85%, 86%, 87%, 88%, 89%, 90%, 91%, 92%, 93%, 94% etc., as well as 81.1%, 81.2%, 81.3%, 81.4%, 81.5%, etc., 82.1%, 82.2%, 82.3%, 82.4%, 82.5%, etc., and so forth.

Reference to an integer with more (greater) or less than includes any number greater or less than the reference number, respectively. Thus, for example, a reference to less than 100, includes 99, 98, 97, etc. all the way down to the number one (1); and less than 10, includes 9, 8, 7, etc. all the way down to the number one (1).

As used herein, all numerical values or ranges include fractions of the values and integers within such ranges and fractions of the integers within such ranges unless the context clearly indicates otherwise. Thus, to illustrate, reference to a numerical range, such as 1-10 includes 1, 2, 3, 4, 5, 6, 7, 8, 9, 10, as well as 1.1, 1.2, 1.3, 1.4, 1.5, etc., and so forth. Reference to a range of 1-50 therefore includes 1, 2, 3, 4, 5, 6, 7, 8, 9, 10, 11, 12, 13, 14, 15, 16, 17, 18, 19, 20, etc., up to and including 50, as well as 1.1, 1.2, 1.3, 1.4, 1.5, etc., 2.1, 2.2, 2.3, 2.4, 2.5, etc., and so forth.

Reference to a series of ranges includes ranges which combine the values of the boundaries of different ranges within the series. Thus, to illustrate reference to a series of ranges, for example, of 1-10, 10-20, 20-30, 30-40, 40-50, 50-60, 60-75, 75-100, 100-150, 150-200, 200-250, 250-300, 300-400, 400-500, 500-750, 750-850, includes ranges of 1-20, 1-30, 1-40, 1-50, 1-60, 10-30, 10-40, 10-50, 10-60, 10-70, 10-80, 20-40, 20-50, 20-60, 20-70, 20-80, 20-90, 50-75, 50-100, 50-150, 50-200, 50-250, 100-200, 100-250, 100-300, 100-350, 100-400, 100-500, 150-250, 150-300, 150-350, 150-400, 150-450, 150-500, etc.

The invention is generally disclosed herein using affirmative language to describe the numerous embodiments and aspects.

A number of embodiments of the invention have been described. Accordingly, the following examples are intended to illustrate but not limit the scope of the invention claimed in any way.

### EXAMPLES

*Example 1 One* of many hypotheses that may account for rAAV vector loss and/or aggregation is that the gel layer may be built up in the TFF apparatus in the non-optimized UF/DF process which may be the cause of the rAAV vector loss. This hypothesis of gel layer built up may also explain the phenomenon that more rAAV vector are lost when rAAV vector was concentrated to high concentration, since more severe gel layer may be built up when vector are more concentrated. Gel layer build up may also cause rAAV aggregation, the phenomenon observed quite often in the UF/DF process, particularly, when the rAAV vectors are concentrated to high concentration.

*Example* 2 In addition to TFF operation parameters, one of many hypotheses is that the formulation may play an important role in reducing gel layer and also preventing rAAV from aggregation, for most the solutions/buffers used in current UF/DF process, the chemicals used in the buffers/solution are salts and chemicals control the pH. If a surfactant was used in the UF/DF solution through the process, it may reduce the association between the rAAV vectors and the association between the rAAV vectors and the surface of the UF/DF apparatus. This in turn could reduce or prevent the gel lay of rAAV vector through the UF/DF process. In addition, the surfactant may further reduce rAAV association by reducing or preventing hydrophobic interaction while going through the UF/DF process, thereby reducing minimizing or preventing rAAV from aggregation. Further hypotheses are that increasing the shear rate and controlling the trans-membrane pressure (TMP) during the UF/DF process may reduce the gel layer built up. Based on all of these hypotheses, a series of studies were undertaken which lead to the particular invention formulations and operation parameters disclosed herein.

*Example 3* According to the invention, new UF/DF operation parameters that reduce, minimize or prevent rAAV from aggregation and increased recovery (overall yield) of rAAV vectors, which is particularly relevant when high titer of rAAV vector preparations are desired. The first operation parameter is to start the UF/DF process with low rAAV titer, especially when the rAAV vector preparations contain high concentration of rAABV, for example, in CsCl gradient centrifugation prior to the UF/DF step. The highly concentrated rAAV in the CsCl was diluted first to a titer of or below 5E+12 vg/ml using diafiltration buffer created containing 10 mM sodium phosphate, 180 mM NaCl and 0.001% of pluronic F-68, at pH 7.3. Operate the DF process using the created diafiltration buffer at flow-rate with shearing rate at about 8,000 sec⁻¹ or higher. Finish the dialfiltration process to achieve efficient buffer exchange, then start the UF process to concentrate the rAAV vector to a desired titer.

One component to have in the newly created UF/DF formulation is Pluronic F-68 which achieved high titer of rAAV preps. Although not wishing to be bound by theory it is believed that this new formulation (diafiltration buffer) reduces gel layer formation.

When the new formulation is combined with the new operation parameters in the UF/DF process, rAAV vector was concentrated to 5E+13 vg per ml without detectable vector aggregation. Furthermore, rAAV vector recovery was substantial, typically at the level of 70% or higher.

## Claims

1. A method for producing recombinant adeno-associated virus (rAAV) vector particles at high recovery or high titer said method comprising the steps of:
(a) harvesting cells and/or cell culture supernatant comprising rAAV vector particles;
(b) optionally concentrating said cells and/or said cell culture supernatant harvested in step (a), optionally via tangential flow filtration, to produce a concentrated harvest;
(c) lysing said harvest produced in step (a) or said concentrated harvest produced in step (b) optionally by microfluidization to produce a lysate;
(d) filtering said lysate produced in step (c) to produce a clarified lysate;
(e) subjecting said clarified lysate produced in step (d) to ion exchange column chromatography to produce a column eluate comprised of purified rAAV vector particles, and optionally concentrating said column eluate by tangential flow filtration to produce a concentrated column eluate;
(f) mixing said column eluate or said concentrated column eluate produced in step (e) with cesium chloride to produce a mixture, and subjecting said mixture to gradient ultracentrifugation to substantially separate *bona fide* rAAV vector particles from empty capsid AAV particles and other AAV vector related impurities;
(g)(1) collecting said *bona fide* rAAV vector particles separated in step (f) and
(g)(2) formulating said collected *bona fide* rAAV vector particles in a buffer with a non-ionic surfactant;
(h) subjecting said *bona fide* rAAV vector particles in step (g)(2) to a buffer exchange by tangential flow filtration to produce an AAV vector formulation; and
(i) filtering said AAV vector formulation produced in step (h) thereby producing a formulation of *bona fide* rAAV vector particles at high recovery or high titer.

2. A method according to claim 1, wherein said buffer of step (g)(2) comprises
(1) sodium chloride, and/or sodium phosphate; or
(2) sodium chloride from a concentration of about 5mM to a concentration of about 500 mM.

3. A method according to claim 1, wherein said non-ionic surfactant of step (g)(2) (1) comprises a polyalkylene glycol;
(2) comprises a block co-polymer comprising at least one polyethylene glycol block and at least one polypropylene glycol block;
(3) comprises an alkyl polyglycoside, cetostearyl alcohol, cetyl alcohol, cocamide DEA, cocamide MEA, decyl glucoside, ethoxylate, IGEPAL CA-630, Isoceteth-20, glucosides, maltosides, monolaurin, nonidet P-40, nonoxynols such as nonoxynol-9, NP-40, octaethylene glycol monododecyl ether, N-octyl beta-D-thioglucopyranosides, octyl glucosides, oleyl alcohol, PEG-10 sunflower glycerides, pentaethylene glycol monododecyl ether, poloxamers such as poloxamer 407, poloxamer 188 (poly(ethylene oxide-co-polypropylene oxide)), polyglycerol polyricinoleate, sorbitans, stearyl alcohol, Triton X-100, or Tween 80;
(4) comprises cetomacrogol 1000, polysorbates such as polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate); polyglycerol polyricinoleate, octadecanoic acid [2-[(2R,3S,4R)-3,4-dihydroxy-2-tetrahydrofuranyl]-2-hydroxyethyl] ester, octadecanoic acid [(2R,3S,4R)-2-[1,2-bis(1-oxooctadecoxy)ethyl]-4-hydroxy-3-tetrahydrofuranyl] ester; C8 to C22 long chain alcohols such as 1-octadecanol, cetylstearyl alcohol, Hexadecan-1-ol and cis-9-octadecen-1-ol; substituted or unsubtituted octylphenol in which the substituents can include a polyethoxyethanol group (e.g., to form octylphenoxypolyethoxyethanol) or any other substituent that will form a non-ionic surfactant with octylphenol; polyethylene glycol monoisohexadecyl ether; dodecanoic acid 2,3-dihydroxypropyl ester; glucosides may include lauryl glucoside, octylglucoside and decyl glucoside; fatty acid amides such as cocamide diethanolamine and cocamide monoethanolamine; or nonionic surfactants that have a hydrophilic polyethylene oxide chain and an aromatic hydrocarbon lipophilic or hydrophilic group, such as Nonoxynol-9 and Triton X-100;
(5) comprises a polyethylene glycol-block-polypropylene glycol-block-polyethylene glycol or a triblock copolymer composed of a central hydrophobic chain of polyoxypropylene (polypropylene oxide)) flanked by two hydrophilic chains of polyoxyethylene (poly(ethylene oxide)); or
(6) has a concentration of about 0.0001 % to a concentration of about 0.1%.

4. A method according to claim 1, wherein
(1) said *bona* fide rAAV vector particles are present in said formulation produced in step (i) at a concentration of about 10¹⁵ particles per mL to about a concentration of 10¹⁸ *bona* fide rAAV vector particles per mL;
(2) the yield of said *bona fide* rAAV vector particles in step (i) is at least about 5×10¹² *bona fide* rAAV vector particles/ml; or
(3) the recovery of said *bona fide* rAAV vector particles in step (i) is at least about 60% of the total rAAV vector particles of step (a).

5. A method according to claim 1, wherein said bona fide rAAV vector particles are derived from an AAV selected from the group consisting of AAV1, AAV2, AAV3, AAV4, AAV5, AAV6, AAV7, AAV8, AAV9, AAV10 and Rh74.

6. A method according to claim 1, wherein
(1) said *bona fide* rAAV particles comprise a transgene that encodes an inhibitory nucleic acid;
(2) said *bona fide* rAAV particles comprise a transgene that encodes a gene product selected from the group consisting of insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), transforming growth factor α (TGFα), platelet-derived growth factor (PDGF), insulin growth factors I and II (IGF-I and IGF-II), TGFβ, activins, inhibins, bone morphogenic protein (BMP), nerve growth factor (NGF), brain-derived neurotrophic factor (BDNF), neurotrophins NT-3 and NT4/5, ciliary neurotrophic factor (CNTF), glial cell line derived neurotrophic factor (GDNF), neurturin, agrin, netrin-1 and netrin-2, hepatocyte growth factor (HGF), ephrins, noggin, sonic hedgehog and tyrosine hydroxylase;
(3) said *bona fide* rAAV particles comprise a transgene that encodes a gene product selected from the group consisting of thrombopoietin (TPO), interleukins (IL1 through IL-17), monocyte chemoattractant protein, leukemia inhibitory factor, granulocyte-macrophage colony stimulating factor, Fas ligand, tumor necrosis factors α and β, interferons α, β, and γ, stem cell factor, flk-2/flt3 ligand, IgG, IgM, IgA, IgD and IgE, chimeric immunoglobulins, humanized antibodies, single chain antibodies, T cell receptors, chimeric T cell receptors, single chain T cell receptors, class I and class II MHC molecules;
(4) said *bona fide* rAAV vector particles comprise a transgene encoding a protein useful for correction of in born errors of metabolism selected from the group consisting of carbamoyl synthetase I, ornithine transcarbamylase, arginosuccinate synthetase, arginosuccinate lyase, arginase, fumarylacetacetate hydrolase, phenylalanine hydroxylase, alpha- 1 antitrypsin, glucose-6-phosphatase, porphobilinogen deaminase, factor V, factor VIII, factor IX, cystathione beta-synthase, branched chain ketoacid decarboxylase, albumin, isovaleryl-coA dehydrogenase, propionyl CoA carboxylase, methyl malonyl CoA mutase, glutaryl CoA dehydrogenase, insulin, beta-glucosidase, pyruvate carboxylate, hepatic phosphorylase, phosphorylase kinase, glycine decarboxylase, RPE65, H-protein, T-protein, a cystic fibrosis transmembrane regulator (CFTR) sequence, and a dystrophin cDNA sequence;
(5) said *bona fide* rAAV vector particles comprise a transgene encoding a protein useful for correction of neurodegenerative diseases; or
(6) said protein is tripeptidyl peptidase 1 (TPP1).

7. A method according to claim 6, part (1), wherein said inhibitory nucleic acid is selected from the group consisting of siRNA, an antisense molecule, miRNA, ribozyme and shRNA.

8. A method according to claim 1, comprising collecting the empty capsid particles separately in step (f).

9. A method according to claim 1, wherein purity of said *bona fide* rAAV vector particles in said filtrate of step (i) is at least about 80%.

10. A method according to claim 1, wherein said empty capsid particles are present in said filtrate of step (i) in an amount of 10% or less.

11. A method according to claim 1, wherein said filtrate of step (i) has no greater than about 10% aggregated rAAV particles.

12. A method according to claim 1, wherein said centrifugation in step (f)
(1) is conducted in a single step; or
(2) is density gradient ultracentrifugation.

13. A method according to claim 1, wherein said column eluate in step (e) is concentrated by tangential flow filtration to produce a concentrated column eluate.

14. A method according to claim 1, further comprising adding a nuclease to the lysate produced in step (c).

15. A method according to any of claims 1-14, wherein the method produces rAAV vector particles having a higher titer than rAAV vector particles produced where a nonionic surfactant is added to the AAV vector formulation after buffer exchange by tangential flow filtration of step (h).

16. A method according to any of claims 1-14, wherein the collected *bona fide* rAAV vector particles of step (g)(1) are diluted to a concentration of less than about 5×10¹² rAAV vector particles/ml, prior to, substantially simultaneously or after step (g)(2).

17. A method according to any of claims 1-14, wherein the tangential flow filtration of step (b), (e) and/or (h) is carried out
(1) at a trans-membrane pressure of between about 2-15 psig; or
(2) at a shear rate greater than about 3000 sec⁻¹.

## Patentansprüche

1. Verfahren zur Herstellung eines rekombinanten adeno-assoziierten Virus-(rAAV) Vektors mit hoher Rückgewinnung oder hohem Titer, wobei das Verfahren die Schritte umfasst:
(a) Ernten der Zellen und/oder des Zellkulturüberstands, umfassend rAAV Vektorpartikel,
(b) gegebenenfalls Konzentrierung der/des im Schritt (a) geernteten Zellen und/oder Zellkulturüberstands, gegebenenfalls über tangentiale Flußfiltration, um eine konzentrierte Ernte zu erhalten,
(c) Lysieren der im Schritt (a) hergestellten Ernte oder der im Schritt (b) hergestellten konzentrierten Ernte, gegebenenfalls durch Mikrofluidisierung, um ein Lysat herzustellen,
(d) Filtrieren des im Schritt (c) hergestellten Lysat, um ein geklärtes Lysat zu erhalten,
(e) Einsetzen des im Schritt (d) hergestellten geklärten Lysats in lonenaustauscher-Säulen-Chromatographie, um ein Säuleneluat herzustellen, das aufgereinigte rAAV Vektorpartikel umfasst, und gegebenenfalls Konzentrierung des Säuleneluats mittels tangentialer Flußfiltration, um ein konzentriertes Säuleneluat zu erhalten;
(f) Vermischen des Säuleneluats oder des konzentrierten Säuleneluats, hergestellt in Schritt (e), mit Cäsiumchlorid um ein Gemisch herzustellen und Einsetzen des Gemischs in einer Gradienten-Ultrazentrifugation, um im Wesentlichen rAAV Vektorpartikel bona fide von leeren Kapsid- rAAV-Partikeln und anderen AW-Vektor verwandten Verunreinigungen abzutrennen;
(g)(1) Sammeln der im Schritt (f) abgetrennten bona fide rAAV Vektorpartikel; und
(g)(2) Formulieren der gesammelten bona fide rAAV Vektorpartikel in einem Puffer mit nicht-ionischen oberflächenaktiven Substanzen;
(h) Einsetzen der bona fide rAAV Vektorpartikel im Schritt (g)(2) in einem Pufferaustauch mittels tangentialer Flußfiltration, um eine rAAV Vektorformulierung herzustellen; und
(i) Filtrieren der im Schritt (h) hergestellten AAV Vektorformulierung, wodurch eine Formulierung der bona fide rAAV Vektorpartikel mit hoher Ausbeute oder hohem Titer hergestellt wird.

2. Verfahren nach Anspruch 1, wobei der Puffer im Schritt (g)(2) umfasst
(a) Natriumchlorid und/oder Natriumphosphat; oder
(b) Natriumchlorid in einer Konzentration von etwa 5mM bis zu einer Konzentration von etwa 500 mM.

3. Verfahren nach Anspruch 1, wobei die nicht-ionische oberflächenaktive Substanz des Schritts (g)(2)
(1) ein Polyalkylenglycol umfasst;
(2) ein Block-Co-Polymer umfasst, umfassend mindestens einen Polyethylenglycol-Block und mindestens einen Polypropylenglycol-Block;
(3) ein Alkylpolyglycosid, Cetostearylalkohol, Cetylalkohol, Cocamid-DEA, Cocamid-MEA, Decylglucosid, Ethoxylat, IGEPAL CA-630, Isoceteth-20, Glucoside, Maltoside, Monolaurin, Nonidet-P-40, Nonoxynole wie Nonoxynol-9, NP-40, Octaethylenglycol-monododecylether, N-Octyl-beta-D-thioglucopyranoside, Octyl-glucoside, Oleylalkohol, PEG-10-Sonnenblumenglyzeride, Pentaethylenglycol-Monododecylether, Poloxamere wie Poloxamer 407, Poloxamer 188 (Poly(ethylen-oxid-co-polypropylenoxid)), Polyglyzerol-Polyricinoleat, Sorbitane, Stearylalkohol, Triton X-100 oder Tween 80 umfasst;
(4) Cetomacrogol 1000, Polysorbate wie Polysorbat 20 (Polyoxyethylen (20)-Sorbitan-Monolaurat), Polysorbat 40 (Polyoxyethylen (20)-Sorbitan-Monopalmitat), Polysorbat 60 (Polyoxyethylen (20)-Sorbitan-Monostearat), Polysorbats 80 (Polyoxyethylen (20)-Sorbitan-Monooleat); Polyglyzerol-Polyricinoleat, Octadecansäure-[2-[(2R,3S,4R)-3,4-Dihydroxy-2-tetrahydrofuranyl]-2-hydroxyethyl]-Ester, Octadecansäure-[(2R,3S,4R)-2-[1,2-Bis(1-oxooctadecoxy)ethyl]-4-hydroxy-3-tetrahydrofuranyl]-Ester; C8 bis C22 langkettige Alkohole wie 1-Octadecanol, Cetylstearyl-Alkohol, Hexadecan-1-ol und Cis-9-octadecen-1-ol; substituiertes oder nicht substituiertes Octylphenol bei dem die Substituenten eine Polyethoxyethanolgruppe (z.B., um Octylphenoxypolyethoxyethanol zu bilden) oder irgendeinen anderen Substituenten, der einen nicht-ionische oberflächenaktiven Stoff mit Octylphenol bilden wird, beinhalten können; Polyethylenglycol-Monoisohexadecylether; Dodecansäure-2,3-Dihydroxypropyl-Ester; Glucoside, die Laurylglucosid, Octylglucosid und Decylglucosid einschließen können; Fettsäureamide wie Cocamid-Diethanolamin und Cocamid-Monoethanolamin; oder nicht-ionische oberflächenaktive Stoffe, die eine hydrophile Polyethylenoxidkette und eine aromatische Kohlenwasserstoff- lipophile oder hydrophile Gruppe haben, wie Nonoxynol-9 und Triton X-100;
(5) ein Polyethylenglycol-Block-Polypropylenglycol-Block-Polyethylenglycol oder ein Triblock-Copolymer zusammengesetzt aus einer zentralen hydrophoben Kette von Polyoxypropylen(poly(propylenoxid)) flankiert von zwei hydrophilen Ketten von Polyoxyethylen(poly(ethylenoxid)) umfasst; oder
(6) eine Konzentration von etwa 0,0001% bis zu einer Konzentration von etwa 0,1% hat.

4. Verfahren nach Anspruch 1, wobei
(1) die bona fide rAAV Vektorpartikel in der im Schritt (i) hergestellten Formulierung in einer Konzentration von etwa 10¹⁵ Partikeln pro mL bis zu einer Konzentration von 10¹⁸ bona fide rAAV Vektorpartikel pro mL anwesend sind;
(2) die Asubeute der bona fide rAAV Vektorpartikel im Schritt (i) mindestes etwa 5×10¹² bona fide rAAV Vektorpartikel/ml ist; oder
(3) die Rückgewinnung der bona fide rAAV Vektorpartikel im Schritt (i) mindestens etwa 60% der gesamten rAAV Vektorpartikel des Schritts (a) sind.

5. Verfahren nach Anspruch 1, wobei die bona fide rAAV Vektorpartikel von einem AAV aus der Gruppe bestehend aus AAV1, AAV2, AAV3, AAV4, AAV5, AAV6, AAV7, AAV8, AAV9, AAV10 und Rh74 abgeleitet sind.

6. Verfahren nach Anspruch 1, wobei
(1) die bona fide rAAV Vektorpartikel ein Transgen umfassen, das eine inhibitorische Nukleinsäure kodiert;
(2) die bona fide rAAV Vektorpartikel ein Transgen umfassen, das ein Genprodukt ausgewählt aus der Gruppe, bestehend aus Insulin, Glucagon Wachstumshormon (GH), Parathormon (PTH), Wachstumshormon-Freisetzungsfaktor (GRF), Follikel stimulierendem Hormon (FSH), luteinisierendem Hormon (LH), humanem Choriongonadotropin (hCG), vaskulärem endothelialem Wachstumsfaktor (VEGF), Angiopoietinen, Angiostatin, Granulozyten-Kolonie-stimulierendem Faktor (GCSF), Erythropoietin (EPO), Bindegewebe-Wachstumsfaktor (CTGF), basischem Fibroblasten-Wachstumsfaktor (bFGF), saurem Fibroblasten-Wachstumsfaktor (aFGF), epidermalem Wachstumsfaktor (EGF), transformierendem Wachstumsfaktor α (TGFα), aus Bluplättchen stammendem Wachstumsfaktor (PDGF), Insulin- Wachstumsfaktoren I and II (IGF-I und IGF-II), TGFβ, Activinen, Inhibinen, Knochenmorphogenem Protein (BMP), Nerven-Wachstumsfaktor (NGF), aus dem Gehirn stammendem neurotrophem Faktor (BDNF), Neurotrophinen NT-3 and NT4/5, ziliarem neurotrophem Faktor (CNTF), aus einer Gliatelllinie stammender neurotropher Faktor (GDNF), Neurturin, Agrin, Netrin-1 and Netrin-2, Hepatozyten-Wachstumsfaktor (HGF), Ephrinen, Noggin, Sonic Hedgehog und Tyrosinhydroxylase umfasst;
(3) die bona fide rAAV Vektorpartikel ein Transgen umfassen, das ein Genprodukt ausgewählt aus der Gruppe bestehend aus Thrombopoietin (TPO), Interleukinen (IL1 bis IL-17), Monozyten-Chemoattraktivumprotein ("monocyte chemoattractant protein"), Leukämie-inhibitorischem Faktor, Granulozyten-Makrophagen-Kolonie stimulierendem Faktor, Fas-Ligand, Tumornekrosefaktoren α and β, Interferone α, β, and γ, Stammzellenfaktor, flk-2/flt3-Ligand, IgG, IgM, IgA, IgD und IgE, chimären Immunoglobulinen, humanisierten Antikörpern, Einzelketten- Antikörpern, T-Zellrezeptoren, chimären T-Zellrezeptoren, Einzelketten-T-Zellrezeptoren, Klasse I und Klasse II MHC-Molekülen umfasst;
(4) die bona fide rAAV Vektorpartikel ein Transgen umfassen, das ein Protein kodiert, das hilfreich ist, um angeborene Fehler des Metabolismus zu korrigieren ausgewählt aus der Gruppe bestehend aus Carbamoylsynthetase I, Ornithintranscarbamylase, Arginosuccinatsynthetase, Arginosuccinatlyase, Arginase, Fumarylacetacetathydrolase, Phenylalaninhydroxylase, Alpha-1-antitrypsin, Glucose-6-phosphatase, Porphobilinogen-Deaminase, Facktor V, Faktor VIII, Faktor IX, Cystathion-beta-synthase, verzweigte Kette Ketosäuredecarboxylase, Albumin, Isovaleryl-coA Dehydrogenase, Propionyl-CoA-carboxylase, Methyl-malonyl-CoA-mutase, Glutaryl-CoA-dehydrogenase, Insulin, Beta-Glucosidase, Pyruvatcarboxylat, hepatischer Phosphorylase, Phosphorylasekinase, Glycindecarboxylase, RPE65, H-Protein, T-Protein, einer zystische Fibrose Transmembranregulator (CFTR)-Sequenz und einer Dystrophin cDNA-Sequenz;
(5) die bona fide rAAV Vektorpartikel ein Transgen umfassen, das ein Protein kodiert, das hilfreich ist, um nuerodegenerative Krankheiten zu korrigieren; oder
(6) das Protein eine Tripeptidyl peptidase 1 (TPP1) ist.

7. Verfahren nach Anspruch 6, Teil (1), wobei die inhibitorische Nukleinsäure ausgewählt ist aus der Gruppe, bestehend aus siRNA, einem Antisinnmolekül, miRNA, Ribozym und shRNA.

8. Verfahren nach Anspruch 1, umfassend das Sammeln der im Schritt (f) abgetrennten leeren Kapsidpartikel.

9. Verfahren nach Anspruch 1, wobei die Reinheit der im guten Glauben rAAV Vektorpartikel im Filtrat von Schritt (i) mindestens etwa 80% ist.

10. Verfahren nach Anspruch 1, wobei die leeren Kapsidpartikel im Filtrat des Schritts (i) in einer Menge von 10% oder weniger vorliegen.

11. Verfahren nach Anspruch 1, wobei das Filtrat von Schritt (i) nicht mehr als etwa 10% aggregierte rAAV Partikel hat.

12. Verfahren nach Anspruch 1, wobei die Zentrifugation im Schritt (f)
(1) in einem einzelnen Schritt vorgenommen wird; oder
(2) eine Dichtegradientenultrazentrifugation ist.

13. Verfahren nach Anspruch 1, wobei das Säuleneluat im Schritt (e) mittels tangentialer Flußfiltration konzentriert wird, um ein konzentriertes Säuleneluat herzustellen.

14. Verfahren nach Anspruch 1, weiterhin umfassend die Zugabe einer Nuklease zu dem im Schritt (c) hergestellten Lysat.

15. Verfahren nach einem der Ansprüche 1-14, wobei das Verfahren rAAV Vektorpartikel herstellt, die einen höheren Titer haben als rAAV Vektorpartikel, die hergestellt wurden, wobei eine nicht-ionische oberflächenaktive Substanz zu der AAV-Vektorformulierung zugegeben wird nach dem Pufferaustausch mittels tangentialer Flußfiltration im Schritt (h).

16. Verfahren nach einem der Ansprüche 1-14, wobei die gesammelten bona fide rAAV Vektorpartikel von Schritt (g)(1) zu einer Konzentration von weniger als etwa 5×10¹² rAAV Vektorpartikel/ml verdünnt werden, vor dem, im Wesentlichen gelichzeitig mit oder nach Schritt (g)(2).

17. Verfahren nach einem der Ansprüche 1-14, wobei die tangentiale Flußfiltration im Schritt (b), (e) und/oder (h) ausgeführt wird
(1) bei einem Trans-Membran-Druck zwischen etwa 2-15 psig; oder
(2) bei einer Scherrate größer als etwa 3000 sec⁻¹.

## Revendications

1. Méthode de production de particules de vecteur de virus adénoassocié recombinant (AAVr) à récupération élevée ou à titre élevé, ladite méthode comprenant les étapes consistant à :
(a) récolter les cellules et/ou le surnageant de culture cellulaire comprenant des particules de vecteur d'AAVr ;
(b) facultativement concentrer lesdites cellules et/ou ledit surnageant de culture cellulaire récoltés à l'étape (a), facultativement par filtration tangentielle, afin de produire une récolte concentrée ;
(c) lyser ladite récolte produite à l'étape (a) ou ladite récolte concentrée produite à l'étape (b) facultativement par microfluidisation afin de produire un lysat ;
(d) filtrer ledit lysat produit à l'étape (c) pour produire un lysat clarifié ;
(e) soumettre ledit lysat clarifié produit à l'étape (d) à une chromatographie en colonne échangeuse d'ions pour produire un éluat de colonne constitué de particules de vecteur d'AAVr purifiées, et facultativement concentrer ledit éluat de colonne par filtration tangentielle, afin de produire un éluat de colonne concentré ;
(f) mélanger ledit éluat de colonne ou ledit éluat de colonne concentré produit à l'étape (e) avec du chlorure de césium pour produire un mélange, et soumettre ledit mélange à une ultracentrifugation en gradient pour séparer sensiblement les particules de vecteur d'AAVr *bona fide* vis-à-vis des particules d'AAV à capside vide et d'autres impuretés liées au vecteur d'AAV ;
(g)(1) collecter lesdites particules de vecteur d'AAVr *bona fide* séparées à l'étape (f) et
(g)(2) formuler lesdites particules de vecteur d'AAVr *bona fide* dans un tampon avec un tensioactif non ionique ;
(h) soumettre lesdites particules de vecteur d'AAVr *bona fide* à l'étape (g)(2) à un échange de tampon par filtration tangentielle afin de produire une formulation de vecteur d'AAV ; et
(i) filtrer ladite formulation de vecteur d'AAV produite à l'étape (h), ce qui permet de produire une formulation de particules de vecteur d'AAVr *bona fide* à une récupération élevée ou un titre élevé.

2. Méthode selon la revendication 1, dans laquelle ledit tampon de l'étape (g)(2) comprend
(1) du chlorure de sodium, et/ou du phosphate de sodium ; ou
(2) du chlorure de sodium d'une concentration d'environ 5 mM à une concentration d'environ 500 mM.

3. Méthode selon la revendication 1, dans laquelle ledit tensioactif non ionique de l'étape (g)(2)
(1) comprend un polyalkylène glycol ;
(2) comprend un copolymère à blocs comprenant au moins un bloc de polyéthylène glycol et au moins un bloc de polypropylène glycol ;
(3) comprend un alkylpolyglycoside, l'alcool cétostéarylique, l'alcool cétylique, le cocamide DEA, le cocamide MEA, le décylglucoside, l'éthoxylate, l'IGEPAL CA-630, l'Isoceteth-20, les glucosides, les maltosides, la monolaurine, le nonidet P-40, les nonoxynols tels que le nonoxynol-9, le NP-40, l'octaéthylène glycol monododécyléther, les N-octyl bêta-D-thioglucopyranosides, les octylglucosides, l'alcool oléylique, les glycérides de tournesol PEG-10, le pentaéthylène glycol monododécyléther, les poloxamères tels que le poloxamère 407, le poloxamère 188 (poly(oxyde d'éthylène-co-polyoxyde de propylène)), le polyglycérol polyricinoléate, les sorbitans, l'alcool stéarylique, Triton X-100 ou Tween 80 ;
(4) comprend le cétomacrogol 1000, les polysorbates tels que le polysorbate 20 (monolaurate de polyoxyéthylène (20) sorbitan), le polysorbate 40 (monopalmitate de polyoxyéthylène (20) sorbitan), le polysorbate 60 (monostéarate de polyoxyéthylène (20) sorbitan), le polysorbate 80 (monooléate de polyoxyéthylène (20) sorbitan) ; le polyglycérol polyricinoléate, le [2-[(2R,3S,4R)-3,4-dihydroxy-2-tétrahydrofuranyl]-2-hydroxyéthyl]ester d'acide octadécanoïque, le [[(2R,3S,4R)-2-[1,2-bis(1-oxooctadécoxy)éthyl-4-hydroxy-3 tétrahydrofuranyl]-ester d'acide octadécanoïque ; les alcools à longue chaîne en C8 à C22 tels que le 1-octadécanol, l'alcool cétylstéarylique, l'hexadécan-1-ol et le cis-9-octadécèn-1-ol ; l'octylphénol substitué ou non substitué dans lequel les substituants peuvent inclure un groupe polyéthoxyéthanol (par exemple pour former de l'octylphénoxypolyéthoxyéthanol) ou tout autre substituant qui formera un tensioactif non ionique avec l'octylphénol ; le polyéthylène glycol monoisohexadécyléther ; le 2,3-dihydroxypropylester d'acide dodécanoïque ; les glucosides peuvent comprendre le laurylglucoside, l'octylglucoside et le décylglucoside ; les amides d'acides gras tels que la cocamide diéthanolamine, et la cocamide monoéthanolamine ; ou les tensioactifs non ioniques qui ont une chaîne d'oxyde de polyéthylène hydrophile et un groupe lipophile ou hydrophile hydrocarboné aromatique, tel que le Nonoxynol-9 et le Triton X-100 ;
(5) comprend un polyéthylène glycol-bloc- polypropylène glycol-bloc-polyéthylène glycol ou un copolymère tribloc constitué d'une chaîne hydrophobe centrale de polyoxypropylène (poly(oxyde de propylène)) flanquée de deux chaînes hydrophiles de polyoxyéthylène (poly(oxyde d'éthylène)) ; ou
(6) a une concentration d'environ 0,0001 % à une concentration d'environ 0,1 %.

4. Méthode selon la revendication 1, dans laquelle
(1) lesdites particules de vecteur d'AAVr *bona fide* sont présentes dans ladite formulation produite à l'étape (i) en une concentration d'environ 10¹⁵ particules par ml à environ une concentration de 10¹⁸ particules de vecteur d'AAVr *bona fide* par ml ;
(2) le rendement desdites particules de vecteur d'AAVr *bona fide* à l'étape (i) est d'au moins environ 5 × 10¹² particules de vecteur d'AAVr *bona fide*/ml ; ou
(3) la récupération desdites particules de vecteur d'AAVr *bona fide* à l'étape (i) est d'au moins environ 60 % des particules totales de vecteur d'AAVr *bona fide* de l'étape (a).

5. Méthode selon la revendication 1, dans laquelle lesdites particules de vecteur d'AAVr *bona fide* sont dérivées d'un AAV choisi dans le groupe consistant en AAV1, AAV2, AAV3, AAV4, AAV5, AAV6, AAV7, AAV8, AAV9, AAV10 et Rh74.

6. Méthode selon la revendication 1, dans laquelle
(1) lesdites particules d'AAVr *bona fide* comprennent un transgène qui code pour un acide nucléique inhibiteur ;
(2) lesdites particules d'AAVr *bona fide* comprennent un transgène qui code pour un produit génique choisi dans le groupe consistant en l'insuline, le glucagon, l'hormone de croissance (GH), l'hormone parathyroïdienne (PTH), le facteur de libération de l'hormone de croissance (GRF), l'hormone folliculo-stimulante (FSH), l'hormone lutéinisante (LH), la gonadotropine chorionique humaine (hCG), le facteur de croissance de l'endothélium vasculaire (VEGF), les angiopoïétines, l'angiostatine, le facteur de stimulation des colonies de granulocytes (GCSF), l'érythropoïétine (EPO), le facteur de croissance du tissu conjonctif (CTGF), le facteur de croissance des fibroblastes basiques (bFGF), le facteur de croissance des fibroblastes acides (aFGF), le facteur de croissance épidermique (EGF), le facteur de croissance transformant α (TGFα), le facteur de croissance dérivé des plaquettes (PDGF), les facteurs de croissance apparentés à l'insuline I et II (IGF-I et IGF-II), le TGFβ, les activines, les inhibines, la protéine morphogénétique osseuse (BMP), le facteur de croissance des nerfs (NGF), le facteur neurotrophique dérivé du cerveau (BNDF), les neurotrophines NT-3 et NT4/5, le facteur neurotrophique ciliaire (CNTF), le facteur neurotrophique dérivé de la lignée des cellules gliales (GDNF), la neurturine, l'agrine, la nétrine-1 et la nétrine-2, le facteur de croissance des hépatocytes (HGF), les éphrines, la noggine, le sonic hedgehog et la tyrosine hydroxylase ;
(3) lesdites particules d'AAVr *bona fide* comprennent un transgène qui code pour un produit génique choisi dans le groupe consistant en la thrombopoïétine (TPO), les interleukines (IL1 jusqu'à IL-17), la protéine chimio-attractive des monocytes, le facteur inhibiteur de la leucémie, le facteur de stimulation des colonies des granulocytes-macrophages, le ligand Fas, les facteurs de nécrose tumorale α et β, les interférons α, β, et γ, le facteur des cellules souches, le ligand flk-2/flt3, l'IgG, l'IgM, l'IgA, l'IgD et l'IgE, les immunoglobulines chimériques, les anticorps humanisés, les anticorps monocaténaires, les récepteurs des lymphocytes T, les récepteurs des lymphocytes T chimériques, les récepteurs de lymphocytes T monocaténaires, les molécules de classe I et de classe II du CMH ;
(4) lesdites particules de vecteur d'AAVr *bona fide* comprennent un transgène qui code pour une protéine utile pour la correction des erreurs héréditaires du métabolisme choisie dans le groupe consistant en la carbamoyle synthétase I, l'ornithine transcarbamylase, l'arginosuccinate synthétase, l'arginosuccinate lyase, l'arginase, la fumarylacétacétate hydrolase, la phénylalanine hydroxylase, l'alpha-1 antitrypsine, la glucose-6-phosphatase, la porphobilinogène désaminase, le facteur V, le facteur VII, le facteur IX, la cystathione bêta-synthase, la kétoacide décarboxylase à chaîne ramifiée, l'albumine, l'isovaléryl-coA déshydrogénase, la propionyl CoA carboxylase, la méthylmalonyl CoA mutase, la glutaryl CoA déshydrogénase, l'insuline, la bêta-glucosidase, le pyruvate carboxylate, la phosphorylase hépatique, la phosphorylase kinase, la glycine décarboxylase, RPE65, la protéine H, la protéine T, une séquence régulatrice transmembranaire de la fibrose kystique (CFTR) et une séquence d'ADNc de dystrophine ;
(5) lesdites particules de vecteur d'AAVr *bona fide* comprennent un transgène qui code pour une protéine utile pour la correction des affections neurodégénératives ; ou
(6) ladite protéine est la tripeptidyl peptidase 1 (TPP1).

7. Méthode selon la revendication 6, partie (1), dans laquelle ledit acide nucléique inhibiteur est choisi dans le groupe consistant en l'ARNsi, une molécule antisens, l'ARNmi, le ribozyme et l'ARNsh.

8. Méthode selon la revendication 1, comprenant l'étape consistant à collecter les particules à capside vide séparément à l'étape (f).

9. Méthode selon la revendication 1, dans laquelle la pureté desdites particules de vecteur d'AAVr bona fide dans ledit filtrat de l'étape (i) est d'au moins environ 80 %.

10. Méthode selon la revendication 1, dans laquelle lesdites particules à capside vide sont présentes dans ledit filtrat de l'étape (i) en une quantité de 10 % ou moins.

11. Méthode selon la revendication 1, dans laquelle ledit filtrat de l'étape (i) n'a pas plus d'environ 10 % de particules d'AAVr agrégées.

12. Méthode selon la revendication 1, dans laquelle ladite centrifugation à l'étape (f)
(1) est réalisée en une seule étape ; ou
(2) est une ultracentrifugation en gradient de densité.

13. Méthode selon la revendication 1, dans laquelle ledit éluat de colonne à l'étape (e) est concentré par filtration tangentielle pour produire un éluat de colonne concentré.

14. Méthode selon la revendication 1, comprenant en outre une étape consistant à ajouter une nucléase au lysat produit à l'étape (c).

15. Méthode selon l'une quelconque des revendications 1 à 14, ladite méthode produisant des particules de vecteur d'AAVr ayant un titre plus élevé que les particules de vecteur d'AAVr produites où un tensioactif non ionique est ajouté à la formulation de vecteur d'AAV après échange tampon par filtration tangentielle de l'étape (h).

16. Méthode selon l'une quelconque des revendications 1 à 14, dans laquelle les particules de vecteur d'AAVr *bona fide* collectées de l'étape (g)(1) sont diluées à une concentration inférieure à environ 5 × 10¹² particules de vecteur d'AAVr/ml avant, sensiblement pendant ou après l'étape (g)(2).

17. Méthode selon l'une quelconque des revendications 1 à 14, dans laquelle la filtration tangentielle de l'étape (b), (e) et/ou (h) est réalisée
(1) à une pression transmembranaire d'entre environ 2-15 psig ; ou
(2) à une vitesse de cisaillement d'environ 3000 sec⁻¹.
